(19) Europäisches Patentamt
European Patent Office
Office européen des brevets

(11) Veröffentlichungsnummer: **0 065 192**
**B1**

# (12) EUROPÄISCHE PATENTSCHRIFT

(45) Veröffentlichungstag der Patentschrift: **01.02.84**

(51) Int. Cl.³: **A 23 K 1/16,** C 07 D 215/58

(21) Anmeldenummer: **82103837.9**

(22) Anmeldetag: **05.05.82**

(54) **Wachstumsförderndes Futtermittel bzw. Tränkflüssigkeit.**

(30) Priorität: **15.05.81 DE 3119384**

(43) Veröffentlichungstag der Anmeldung: **24.11.82 Patentblatt 82/47**

(45) Bekanntmachung des Hinweises auf die Patenterteilung: **01.02.84 Patentblatt 84/5**

(84) Benannte Vertragsstaaten: **DE FR GB IT NL**

(56) Entgegenhaltungen:
**EP - A - 0 012 725**
**DE - C - 455 387**

(73) Patentinhaber: **BASF Aktiengesellschaft, Carl-Bosch-Strasse 38, D-6700 Ludwigshafen (DE)**

(72) Erfinder: **Lechtken, Peter, Dr., Ludwigshafener Strasse 6B, D-6710 Frankenthal (DE)**
Erfinder: **Nuerrenbach, Axel, Dr., Koenigsberger Strasse 7, D-6718 Gruenstadt (DE)**
Erfinder: **Schole, Juergen, Dr., Weissdornweg 19, D-3002 Wechmark (Mellendorf) (DE)**

**EP 0 065 192 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Wachstumsförderndes Futtermittel bzw. Tränkflüssigkeit

Die Erfindung betrifft von antibiotischen Nebenwirkungen praktisch freie wachstumsfördernde Futtermittel bzw. Tränkflüssigkeiten für Haus- und Nutztiere, insbesondere Geflügel und Schweine.

Es sind eine Reihe von Stoffen in Gebrauch, die das Wachstum von Nutztieren fördern bzw. die Futterverwertung verbessern. Die wichtigsten Vertreter dieser Stoffe lassen sich beispielsweise aus dem Kapitel »Zusatzstoffe in Alleinfuttermitteln« aus dem deutschen Futtermittelrecht entnehmen. Für die Wirkung dieser Stoffe ist ihre antibiotische oder antibakterielle Wirkung nicht unerheblich.

Wegen der Resistenzbildung bestand jedoch die Aufgabe, ein wachstumsförderndes oder die Futterverwertung verbesserndes Futtermittel ohne (oder praktisch ohne) antibiotische oder antibakterielle Wirkung vorzuschlagen.

Es wurde nun gefunden, daß Tierfuttermittel, die in geringen Mengen, d. s. 0,5 bis 500 ppm, Verbindungen der Formeln Ia, b enthalten,

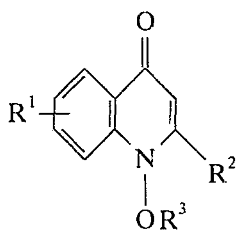
(Ia) oder
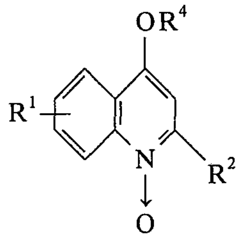
(Ib)

in der

$R^1$ Wasserstoff, einen niedrigmolekularen Alkyl- oder Alkoxyrest oder Halogen,
$R^2$ Wasserstoff oder einen niedrigmolekularen Alkylrest und
$R^3$ und $R^4$ Wasserstoff, einen niedrigmolekularen aliphatischen oder einen araliphatischen Rest, vorzugsweise einen Methyl-, Ethyl- oder Benzylrest bedeuten,

einen wachstumsfördernden Effekt für Tiere entfalten, d. h. zu einer rascheren Gewichtszunahme der Tiere führen bzw. die Futterverwertung durch das Tier verbessern. In der angewandten wirksamen Konzentration haben diese Verbindungen keine antibiotische oder antibakterielle Wirkung.

Die Verbindungen der Formeln Ia, b können den Tieren mit dem üblichen Futter oder insbesondere bei Derivaten mit $R^3$ oder $R^4 \neq H$ auch mit dem Trinkwasser verabreicht werden. Zur Herstellung solcher Gemische eignen sich z. B. Vorgemische oder Konzentrate (bzw. Futterzusatzmittel) mit einem Gehalt von 1 bis 50% an Verbindungen der Formeln Ia, b. Die Futtervorgemische, Futtermittelkonzentrate und Futtermittel können als Träger jede Substanz pflanzlichen oder tierischen Ursprungs, die der Fütterung dient, enthalten. Zweckmäßige Trägersubstanzen sind Weizengrieß, Gerste, Roggen, Hafermehl, Reiskleie, Weizenkleie, Sojamehl, Maiskeimlingsmehl, Knochenmehl, Luzernenmehl, Sojagrieß, Fleischmehl und Fischmehl sowie ihre Gemische.

Als Hilfsstoffe können die Futtervorgemische, Futtermittelkonzentrate und Futtermittel vorteilhaft Siliciumdioxid, Netzmittel, Antioxidationsmittel, Stärke, Dicalciumphosphat, Calciumcarbonat und/oder Sorbinsäure enthalten. Die Netzmittel können z. B. nicht-toxische Öle, vorteilhaft Soja-, Mais- oder Mineralöl, sein. Als vorteilhafte Netzmittel haben sich auch die verschiedenen Alkylenglykole erwiesen. Die Stärke kann vorteilhaft Mais-, Weizen- oder Kartoffelstärke sein. Gegebenenfalls können die Verbindungen der Formeln Ia, b auch auf Trägermaterialien aufgezogen, mit Gelatine umhüllt oder microverkapselt werden. Diese Techniken sind dem Fachmann geläufig.

Durch Einmischen entsprechender Mengen der erfindungsgemäßen Konzentrate in die für jede Tierart spezifische Futtermittel-Mischung kann jeweils die optimale Wirkstoffmenge für jede Tierart eingestellt werden.

Die Verbindungen der Formeln Ia, b können im Falle von $R^3$ bzw. $R^4 = H$ in einem tautomeren Gleichgewicht vorliegen.

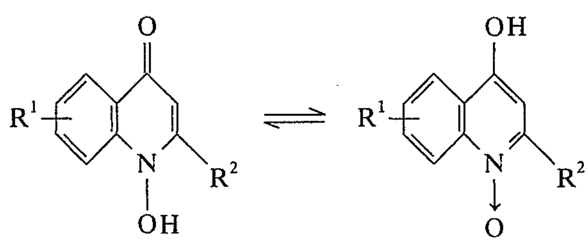

Einige Verbindungen der allgemeinen Formeln Ia, b sind bereits bekannt. So erhielten Gabriel und Gerhard (Chem. Ber. 54 (1921), 1071, 1072) die Verbindung der Formel II und Cornforth u. James

(Biochem. J. 63, 124 (1956)) die Verbindungen der Formel III.

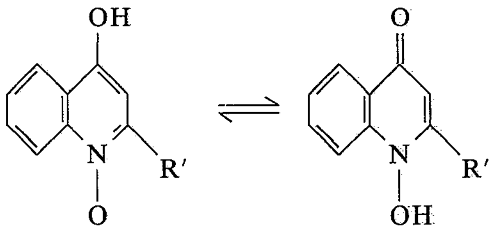

II: $R' = CH_3$
III: $R' = C_7$ bis $C_{11}$-Alkyl

Hingegen sind die Verbindungen der allgemeinen Formel IVa, b

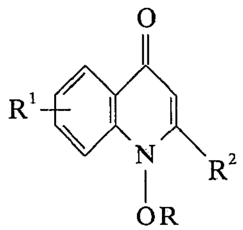
(IVa) und
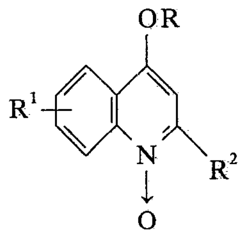
(IVb)

in der $R^1$, $R^2$ die oben angegebenen Bedeutungen haben und R einen Methyl-, Ethyl- oder Benzylrest bedeutet, bisher nicht beschrieben worden. Diese neuen Vebindungen zeichnen sich durch besonders starke wachstumsfördernde Wirkung aus.

Die Verbindungen der Formeln Ia, b kann man beispielsweise aus den entsprechend substituierten 4-Hydroxy-chinolinen der Formel V herstellen

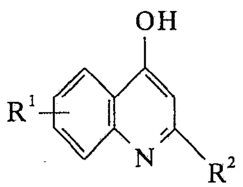
(V)

in der $R^1$ und $R^2$ die oben angegebenen Bedeutungen haben. Diese Hydroxychinoline erhält man in an sich bekannter Weise, z. B. durch Kondensation von entsprechenden Anilinen mit $\beta$-Ketoestern nach Gleichung (1) und (2), wie dies in der deutschen Patentschrift 455 387 beschrieben ist.

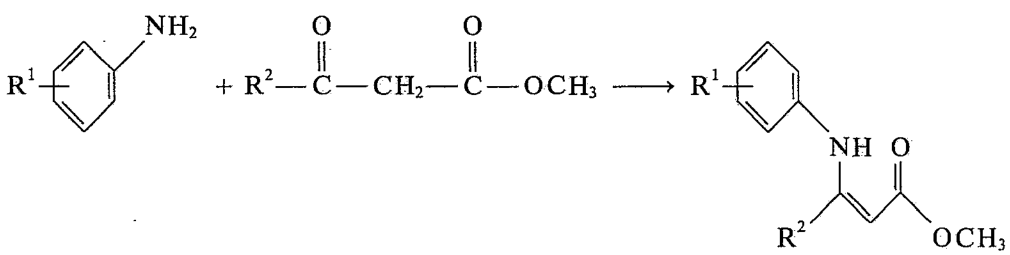
(1)

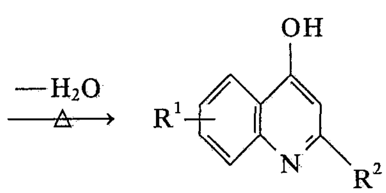

(2)

Die 4-Hydroxychinoline bzw. die durch Alkylierung erhaltenen 4-Alkoxy- und 4-Benzyloxychinoline können durch Persäuren oder $H_2O_2$ in essigsaurer Lösung, bevorzugt durch Peressigsäure, Perphthalsäure, m-Chlor-perbenzoesäure mit oder ohne Molybdän- oder Wolframsäure als Katalysator, zu den entsprechenden Verbindungen der Formeln Ia, b oxidiert werden. Im Falle der Oxidation der 4-Hydroxychinoline wird die 4-Hydroxygruppe zweckmäßig durch Veresterung mit Essigsäure, Chlorkohlensäureester oder Benzoesäure geschützt. Nach erfolgter Oxidation kann diese Schutzgruppe leicht durch Rühren mit verdünnter Natron- oder Kalilauge bei Temperaturen von 0 bis 60°C, vorzugsweise

10 bis 30°C, entfernt werden. Durch anschließendes Ansäuern wird das Produkt in guter Reinheit ausgefällt.

Eine weitere Methode der Herstellung folgt der Vorschrift von Gabriel und Gerhard in Chem. Ber. 54 (1921), 1071, 1072, die durch Reduktion von o-Nitrobenzoylaceton das 4-Hydroxy-chinaldin-N-oxid erhielten.

Die erfindungsgemäßen Verbindungen der Formel Ia mit $R^3 \neq H$ werden aus der Vorstufe ($R^3 = H$) durch Alkylierung mit Dialkylsulfaten oder Dialkylsulfiten oder Alkyl- bzw. Benzylhalogeniden erhalten. Als Lösungsmittel dienen polare Lösungsmittel wie Tetrahydrofuran, Dioxan, Ether oder Dimethylformamid, gegebenenfalls in Gegenwart von Basen und bis zu 50% Wasser.

Im einzelnen seien beispielsweise genannt:

4-Hydroxychinaldin-N-oxid, 6-Methyl-4-hydroxychinaldin-N-oxid,
6-Ethoxy-4-hydroxychinaldin-N-oxid, 6-Chlor-4-hydroxychinaldin-N-oxid,
8-Methyl-4-hydroxychinaldin-N-oxid, N-Methoxy-chinaldon-4,
6-Methyl-N-methoxy-chinaldon-4, 4-Methoxy-chinaldin-N-oxid,
6-Ethoxy-4-methoxy-chinaldin-N-oxid, 4-Benzyloxy-chinaldin-N-oxid,
6-Chloro-4-methoxy-chinaldin-N-oxid, 2-Butyl-4-hydroxy-chinolin-N-oxid.

## Beispiel 1

80 g 4-Hydroxychinaldin (0,5 Mol) werden mit 27 g (0,5 Mol) $NaOCH_3$ in 400 ml trockenem Methanol in das Na-Salz überführt. Nach Entfernen des Methanols durch Abdampfen im Wasserstrahlvakuum wird das trockene Na-Salz in 700 ml trockenem Toluol suspendiert und bei 80°C langsam mit 54 g (0,5 Mol) Chlorameisensäureethylester versetzt. Nach 2 Stunden wird das Lösungsmittel im Wasserstrahlvakuum abdestilliert, der Rückstand 30 min in 600 ml Methylenchlorid aufgerührt und abgesaugt.

Das Filtrat versetzt man bei 15°C portionsweise mit 82,5 g (0,5 Mol) 85%iger m-Chlorperbenzoesäure. Nach einer Stunde wird mit kalter verdünnter Sodalösung und mit Wasser ausgeschüttelt, die organische Phase getrocknet und im Wasserstrahlvakuum eingeengt. Den Rückstand nimmt man in 200 ml Ethanol (50%ig) auf und versetzt bei Raumtemperatur mit einer Lösung von 16 g KOH in 200 ml Ethanol (50%ig). Nach 30 min wird auf das halbe Volumen eingeengt, das Produkt durch Ansäuern mit 2 n HCl auf pH 4 ausgefällt. Man erhält 47 g bis 60 g Rohprodukt. Dies kann gegebenenfalls aus Methanol umkristallisiert werden zum reinen 4-Hydroxychinalidin-N-oxid. Fp.: 248 bis 250°C.

## Beispiel 2

14 g (0,08 Mol) 4-Hydroxychinaldin-N-oxid werden in 400 ml Dioxan suspendiert und mit 4,8 g (0,12 Mol) NaOH in 80 ml $H_2O$ versetzt. Man rührt bei 50°C und tropft 13,2 g (0,116 Mol) Dimethylsulfat in 80 ml Dioxan zu. Nach 2 Stunden wird die Lösung im Wasserstrahlvakuum eingeengt, der Rückstand in 20 ml Wasser aufgenommen und mit conc. HCl auf pH 1 angesäuert. Nach Absaugen und Trocknen erhält man 14 g Rohprodukt, das aus Wasser unter Zusatz von A-Kohle umkristallisiert werden kann.

Ausbeute: 6 bis 8 g N-Methoxy-chinaldon-4, Fp.: 186 bis 188°C/Zersetzung.

## Beispiel 3

31 g 4-Methoxychinaldin (hergestellt aus 4-Hydroxy-chinaldin nach M. Maurin, A. Ch. 4 [11], 301, 335 (1935)) werden bei Raumtemperatur in 80 ml Chloroform aufgenommen und unter Rühren portionsweise mit 42 g m-Chlorperbenzoesäure (85%ig) versetzt. Man erwärmt 2 h auf 40°C, kühlt auf 20°C ab und stellt mit ca. 9 g NaOH in 50 ml $H_2O$ auf pH 6—7 ein. Die organische Phase wird abgetrennt, mit $Na_2SO_4$ getrocknet, filtriert und eingeengt. Es verbleibt 34 g Rohprodukt, das aus Toluol unter Zusatz von wenig festem KOH umkristallisiert wird. Man erhält 12 g reines 4-Methoxy-chinaldon-N-oxid. Fp.: 131—132°C.

## Beispiel 4

In Analogie zu den Angaben der Beispiele 1—3 werden die folgenden weiteren Verbindungen erhalten.

Tabelle

| | Fp. [°C] | Analyse | | |
|---|---|---|---|---|
| 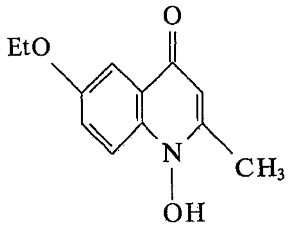 | 248–250 | C 65,74<br>C 65,7 | H 5,98<br>H 6,0 | N 6,39 ber.<br>N 6,4 gef. |
| 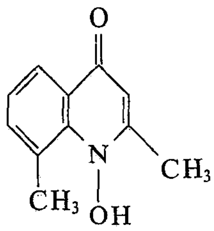 | 253–254 | C 69,83<br>C 70,0 | H 5,86<br>H 6,1 | N 7,40 ber.<br>N 7,7 gef. |
| 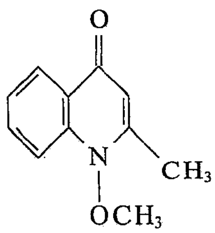 | 186–188 (Zers.) | C 69,84<br>C 70,1 | H 5,86<br>H 6,0 | N 7,40 ber.<br>N 7,5 gef. |
| 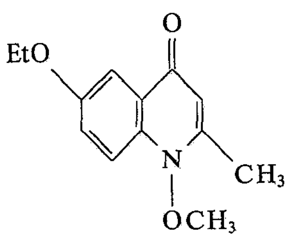 | | keine Analyse, sehr hygroskopisch | | |
| 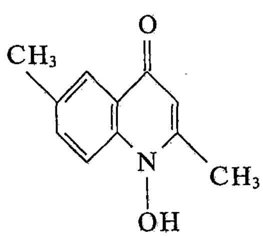 | 248–252 | C 69,83<br>C 70,3 | H 5,86<br>H 5,9 | N 7,40 ber.<br>N 7,6 gef. |
| 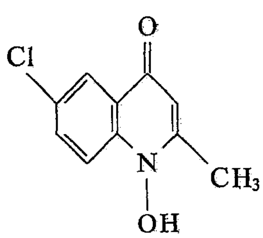 | 265–267 (Zers.) | C 57,28<br>C 57,1 | H 3,82<br>H 3,8 | N 6,68 ber.<br>N 6,7 gef. |
| 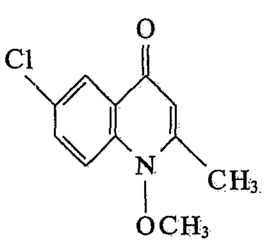 | 128–133 | C 59,06<br>C 59,3 | H 4,47<br>H 4,7 | N 6,26 ber.<br>N 6,3 gef. |

## Beispiel 5

Futter-Zusatzmittel mit einem Gehalt an Verbindungen der Formel Ia, b als Wirkstoff können folgendermaßen formuliert werden:

Aus 10 g Wirkstoff der Formel Ia, b und 90 g Maisstärke wird durch Vermahlen ein 10%iges Konzentrat hergestellt, das durch weiteres Vermahlen mit 900 g Weizengrießkleie zu einer 1%igen Vormischung verdünnt wird.

Durch Vermischen von 10 g dieser Vormischung mit 990 g eines handelsüblichen Standardfutters für Schweine, Geflügel oder Wiederkäuer erhält man ein Futtermittel mit einem Gehalt an 100 ppm Wirkstoff.

## Beispiel 6

Es wurde für Frischlinge eine Vormischung (Premix) der folgenden Zusammensetzung bereitet:

| Bestandteile | Menge |
|---|---|
| Vitamin A | 1 200 000 IE |
| Vitamin $D_3$ | 300 000 IE |
| Vitamin E | 2 000 IE |
| Vitamin $B_2$ | 600 mg |
| Vitamin $B_3$ | 2 000 mg |
| Vitamin $B_{12}$ | 5 mg |
| Nicotinsäure (Niacin) | 3 000 mg |
| Cholinchlorid | 40 000 mg |
| N-Methoxy-chinaldon-4 (aus Beispiel 2) | 1 000 mg |
| Butylhydroxytoluol (Antioxidationsmittel) | 30 000 mg |
| Spurenelemente | |
| Mangan | 6 000 mg |
| Eisen | 10 000 mg |
| Zink | 15 000 mg |
| Kupfer | 30 000 mg |
| Iod | 100 mg |
| 2mal gemahlene Kleie zum Auffüllen auf | 1 000 g |

Diese Vitamin- und Spurenelementenvormischung wurde dem Basisfuttermittel in einer Menge von 0,5 kg je 100 kg zugesetzt.

## Beispiel 7

0,5 kg der nach dem Beispiel 6 bereiteten Vormischung wurde einem Grundfuttermittel der folgenden Zusammensetzung zugemischt:

| Bestandteil | Menge |
|---|---|
| Mais | 25,0 kg |
| Weizen | 34,0 kg |
| Extrahierte Soja | 18,0 kg |
| Milchpulver | 9,9 kg |
| Fischmehl | 4,0 kg |
| Futterhefe | 2,0 kg |
| Fettpulver | 3,4 kg |
| Mineralvormischung (Dicalciumphosphat/Monocalciumphosphat/Calciumcarbonat = 55/40/5) | 1,8 kg |
| Futterkalk | 1,0 kg |
| Natriumchlorid von Futterqualität | 0,4 kg |
| Vormischung des Beispiels 6 | 0,5 kg |
| Gesamtgewicht | 100,0 kg |

Der Wirkstoffgehalt des so erhaltenen Frischlingfuttermittels betrug 5 ppm = 0,0005 Gew.-%.

## Beispiel 8

Die Wirksamkeit der erfindungsgemäßen Futtermittel hinsichtlich einer Verbesserung der Wachstumsleistung und Futterverwertung sei durch einen Fütterungsversuch an Ratten illustriert:

Die Versuchstiere, männliche Sprague-Dawley-Ratten (Firma Jautz, Kisslegg) wurden jeweils zu dritt in Makrolonkäfigen gehalten und mit handelsüblichem Versuchstierfutter (Eggersmann, Rinteln) gefüttert. Futter und Wasser standen ohne Mengenbegrenzung zur Verfügung. Die Ergebnisse enthält Tabelle 2.

Tabelle 2

| Substanz | Konz. im Futter | Tierzahl | Körpergewicht/Tier 0. Tag | 21. Tag | Gewichtszunahme in % der Kontrolle | Futterverwertung |
|---|---|---|---|---|---|---|
| Kontrolle | – | 18 | 74,9 ± 2,9 | 209 ± 12,2 | 100 | 2,67 |
| 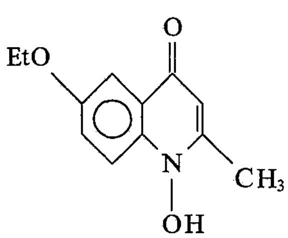 | 10 ppm | 9 | 74,8 ± 2,9 | 215,6 ± 12,3 | 104,6 | 2,59 |
| 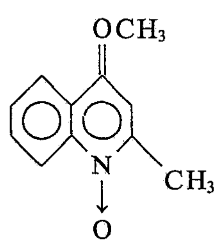 | 5 ppm | 9 | 74,8 ± 2,1 | 219,6 ± 13,9 | 107,6 | 2,55 |

**Patentansprüche**

1. Wachstumsförderndes Futtermittel oder Tränkflüssigkeit, enthaltend neben der Hauptmenge eines üblichen Futtermittelvorgemisches, Futtermittelkonzentrats oder Futtermittels bzw. Wasser geringe Mengen an Verbindungen der Formeln Ia, b

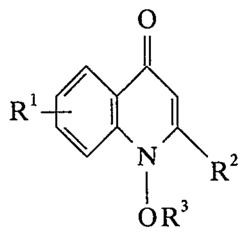 (Ia) oder 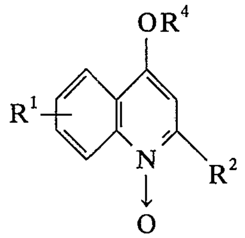 (Ib)

in denen

$R^1$ Wasserstoff, einen niedrigmolekularen Alkyl- oder Alkoxyrest oder Halogen,
$R^2$ Wasserstoff oder einen niedrigmolekularen Alkylrest und
$R^3$ und $R^4$ Wasserstoff, einen niedrigmolekularen aliphatischen oder einen araliphatischen Rest, bedeuten.

2. Futtermittel oder Tränkflüssigkeit gemäß Anspruch 1, enthaltend 0,5 bis 500 ppm der Verbindungen der Formeln Ia, b gemäß Anspruch 1.
3. Futtermittel oder Tränkflüssigkeit gemäß Anspruch 1, enthaltend die Verbindungen

4-Hydroxychinaldin-N-oxid, 6-Methyl-4-hydroxy-chinaldin-N-oxid, 6-Ethoxy-4-hydroxychinaldin-N-oxid, 6-Chlor-4-hydroxy-chinaldin-N-oxid, 8-Methyl-4-hydroxychinaldin-N-oxid, N-Methoxy-chinaldon-4, 6-Methyl-N-methoxy-chinaldon-4, 4-Methoxy-chinaldin-N-oxid, 6-Ethoxy-4-methoxy-chinaldin-N-oxid, 4-Benzyloxy-chinaldin-N-oxid, 6-Chloro-4-methoxy-chinaldin-N-oxid, 2-Butyl-4-hydroxychinolin-N-oxid.

4. Verwendung von Verbindungen der Formeln Ia, b gemäß Anspruch 1 als Zusatz in geringen Mengen zu Futtermittelvorgemischen, Futtermittelkonzentraten oder Futtermitteln bzw. Tränkflüssigkeiten.
5. Verwendung von Verbindungen der Formel I gemäß Anspruch 1 als Zusatz zu Futtermittelvorgemischen, Futtermittelkonzentraten oder Futtermitteln oder Tränkflüssigkeiten in einer Menge von 0,5 bis 500 ppm.
6. Verbindungen der Formeln IVa, b

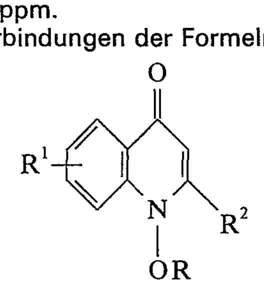 (IVa) und 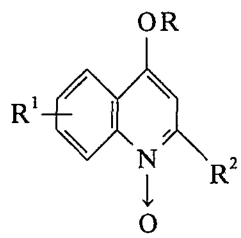 (IVb)

in denen $R^1$ ein Wasserstoffatom, einen niedrigmolekularen Alkyl- oder Alkoxyrest oder Halogen, $R^2$ ein Wasserstoffatom oder einen niedrigmolekularen Alkylrest und R einen Methyl-, Ethyl- oder Benzylrest bedeutet.

**Claims**

1. A growth-promoting feed or drink which in addition to a conventional feed premix, feed concentrate or feed, or water, as the main constituent, contains a small amount of a compound of the formula Ia or Ib

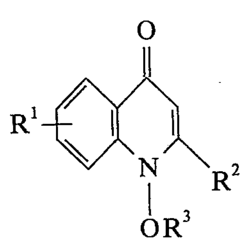 (Ia)

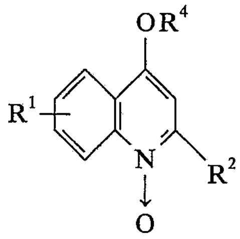

(Ib)

where $R^1$ is hydrogen, low molecular weight alkyl or alkoxy, or halogen, $R^2$ is hydrogen or low molecular weight alkyl and $R^3$ and $R^4$ are hydrogen, a low molecular weight aliphatic radical or an araliphatic radical.

2. A feed or drink as claimed in claim 1, which contains from 0.5 to 500 ppm of a compound of the formula Ia or Ib of claim 1.

3. A feed or drink as claimed in claim 1, which contains

4-hydroxyquinaldine-N-oxide, 6-methyl-4-hydroxyquinaldine-N-oxide,
6-ethoxy-4-hydroxyquinaldine-N-oxide, 6-chloro-4-hydroxyquinaldine-N-oxide,
8-methyl-4-hydroxyquinaldine-N-oxide, N-methoxy-quinald-4-one,
6-methyl-N-methoxy-quinald-4-one, 4-methoxy-quinaldine-N-oxide,
6-ethoxy-4-methoxy-quinaldine-N-oxide, 4-benzyloxy-quinaldine-N-oxide,
6-chloro-4-methoxy-quinaldine-N-oxide or 2-butyl-4-hydroxyquinoline-N-oxide.

4. The use of a compound of the formula Ia or Ib of claim 1 as an addition in a small amount to feed premixes, feed concentrates, feeds or drinks.

5. The use of a compound of the formula Ia or Ib of claim 1 as an addition to feed premixes, feed concentrates, feeds or drinks, in an amount of from 0.5 to 500 ppm.

6. A compound of the formula IVa or IVb

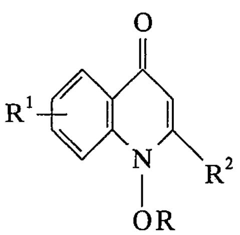

(IVa)

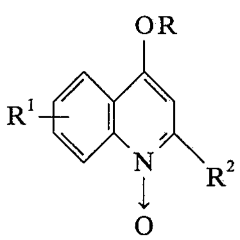

(IVb)

where $R^1$ is hydrogen, low molecular weight alkyl or alkoxy or halogen, $R^2$ is hydrogen or low molecular weight alkyl and R is methyl, ethyl or benzyl.

**Revendications**

1. Aliment ou boisson favorisant la croissance, caractérisé en ce qu'il contient, outre unę majeure partie d'un pré-mélange d'aliments, d'un concentré pour produits alimentaires ou d'un aliment usuel et d'eau, de petites quantités de composés de formules Ia, b

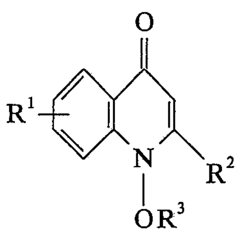

(Ia)

ou

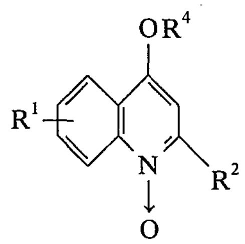

(Ib)

dans lesquelles

$R^1$ représente un hydrogène, un radical alkyle ou alcoxy inférieur ou un halogène,
$R^2$ représente un hydrogène ou un radical alkyle inférieur et
$R^3$ et $R^4$ représentent un hydrogène, un radical aliphatique ou araliphatique inférieur.

2. Aliment ou boisson selon la revendication 1, caractérisé en ce qu'il contient entre 0,5 et 500 ppm des composés de formules Ia, b selon la revendication 1.

3. Aliment ou boisson selon la revendication 1, caractérisé en ce qu'il contient le composé

N-oxyde de 4-hydroxyquinaldine, N-oxyde de 6-méthyl-4-hydroxy-quinaldine,
N-oxyde de 6-éthoxy-4-hydroxyquinaldine, N-oxyde de 6-chloro-4-hydroxy-quinaldine,
N-oxyde de 8-méthyl-4-hydroxy-quinaldine, N-méthoxy-quinaldone-4,
6-méthyl-N-méthoxy-quinaldone-4, N-oxyde de 4-méthoxy-quinaldine,
N-oxyde de 6-éthoxy-4-méthoxy-quinaldine, N-oxyde de 4-benzyloxyquinaldine,
N-oxyde de 6-chloro-4-méthoxy-quinaldine ou N-oxyde de 2-butyl-4-hydroxyquinoléïne.

4. Utilisation de composés des formules Ia, b selon la revendication 1 comme additifs en petites quantités à des pré-mélanges d'aliments, des concentrés pour produits alimentaires, des aliments ou des boissons.

5. Utilisation de composés de formule I selon la revendication 1 comme additifs à des pré-mélanges d'aliments, des concentrés pour produits alimentaires, des aliments ou des boissons, dans une proportion de 0,5 à 500 ppm.

6. Composés de formules IVa, b

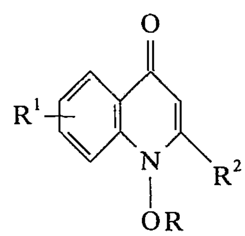

(IVa)

et

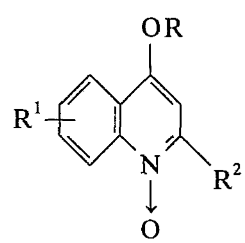

(IVb)

dans lesquelles $R^1$ est un atome d'halogène, un radical alkyle ou alcoxy inférieur ou un halogène, $R^2$ est un atome d'hydrogène ou un radical alcoyle inférieur et R est un radical méthyle, éthyle ou benzyle.